# EUROPÄISCHE PATENTSCHRIFT

(11) **EP 2 240 198 B1**
(45) Veröffentlichungstag und Bekanntmachung des Hinweises auf die Patenterteilung: **14.12.2011**
(21) Anmeldenummer: 09710075.4
(22) Anmeldetag: 13.02.2009
(51) Int. Cl.: A61K 38/27, A61K 38/48, A61P 3/02, A61P 5/00, A61K 9/00

(54) **ZUSAMMENSETZUNG ZUR AUFNAHME ÜBER MUKÖSES GEWEBE**
COMPOSITION FOR ABSORPTION THROUGH MUCOUS TISSUE
COMPOSITION DESTINÉE À ÊTRE DÉPOSÉE SUR UN TISSU MUQUEUX

(30) Priorität: 13.02.2008 AT 2382008
(43) Veröffentlichungstag der Anmeldung: 20.10.2010
(73) Patentinhaber: Hernuss, Peter, 1180 Wien (AT)
(72) Erfinder: Hernuss, Peter, 1180 Wien (AT)
(74) Vertreter: Babeluk, Michael
(86) Internationale Anmeldenummer: PCT/EP2009/051682
(87) Internationale Veröffentlichungsnummer: WO 2009/101165

(56) Entgegenhaltungen:
- WO-A-02/058735
- WO-A-2004/113522
- WO-A-2006/009825
- WO-A-2007/067964
- US-A1- 2007 009 583
- US-A1- 2007 014 735

## Beschreibung

Die Erfindung betrifft eine Zusammensetzung zur Aufnahme über muköses Gewebe, enthaltend zumindest ein mittels Proteinasen spaltbares Hormon sowie zumindest eine Proteinase oder eine Proteinasenmischung, wobei das zumindest eine Hormon in der Zusammensetzung getrennt von der Proteinase oder Proteinasenmischung vorliegt, das neben einer allgemeinen Verbesserung des Wohlbefindens Stoffe zur Anregung der Hormonproduktion im Körper bereitstellt, sowie deren Verwendung zur Herstellung eines Nahrungsergänzungsmittels und einen Kit zur Verabreichung der Zusammensetzung.

Nahrungsergänzungsmittel erfreuen sich immer größerer Beliebtheit. Ihre Aufgabe ist es, eine gesunde Lebensweise zu unterstützen oder einen erhöhten Bedarf an bestimmten Nährstoffen aufgrund einer anspruchsvollen Lebensweise oder Mängel in der Ernährung auszugleichen.

Ebenso haben sie zum Teil eine sogenannte "Anti-Aging" - Wirkung, und es gibt eine erhöhte Nachfrage an derartigen Anti-Aging-Produkten, die eine Verzögerung des Alterungsprozesses sowie eine erhebliche Verbesserung der Lebensqualität im Alter bewirken.

Es ist beispielsweise bekannt, dass mit dem Alter der Wachstumshormonspiegel abnimmt. Bei Unterstützung der Wachstumshormonsynthese ab etwa dem 40. Lebensjahr wurde eine erheblich Verbesserung der Leistungsfähigkeit und der physiologischen Abläufe festgestellt.

Somatotropin (hGH, hypophyseal oder human growth hormone) ist ein im Hypophysen-Vorderlappen gebildetes Hormon, welches sowohl für den Wachstumsprozess von Kindern als auch im Erwachsenenalter für den Ablauf zahlreicher Körperfunktionen verantwortlich ist. Ein Fehlen des Somatotropins in der Jugend bewirkt Wachstumsstillstand (Zwergwuchs), eine erhebliche Überproduktion hingegen Akromegalie (Riesenwuchs; verstärktes Wachstum der Akren = Hände und Füße). Das Wachstumshormon des Menschen ist ein einzelnes Polypeptid mit einem Molekulargewicht von 21.500 Dalton und besteht aus 191 Aminosäuren bekannter Sequenz unter anderem mit einer Anzahl von Disulfid-Brücken.

Bei der oralen Einnahme von hGH wird dieses im Magen sofort zerstört und hat daher keinerlei Wirkung im menschlichen Körper. Eine Aufnahme eines kompletten Wachstumshormonmoleküls über die Schleimhäute ist nicht möglich, weil es aufgrund seiner Molekülgröße diese nicht durchdringen kann. Daher erfolgt die Verabreichung von Somatotropin für gewöhnlich mittels Injektionen. Ähnlich verhält es sich mit anderen Peptidhormonen, deren Molekülgröße eine Aufnahme über die Schleimhäute unmöglich macht, und die bei oraler Einnahme von der Magensäure denaturieren und damit unwirksam werden. Kürzere Polypeptide hingegen können grundsätzlich dem Organismus über die Haut oder Schleimhaut zugeführt werden. Diese Zufuhr ist durch verschiedene Faktoren, insbesondere durch die Molekülgröße des Peptids limitiert. So stellt bei Polypeptiden eine Molekülgröße mit einer Länge von etwa 80 Aminosäuren und einem Molekulargewicht von etwa 10.000 Dalton eine derartige Obergrenze für die Aufnahme über die Schleimhaut dar.

In der WO 2007/067964 A wird eine pharmazeutische Zusammensetzung mit einer therapeutische Menge an Exendin zur intranasalen Applikation zur Gewichtskontrolle beschrieben. In der US 2007/0009538 A1 ist ein Wundpflaster mit Liposomen beschrieben. Die US 2007/0014735 A1 offenbart eine Zusammensetzung mit einem inaktivierten bioaktiven Peptid sowie einem quaternären Ammoniumsalz zur Verbesserung der mukosalen Aufnahme des Peptides. In der WO 2002/058735 A1 schließlich wird ein Verfahren zur Herstellung von Produkten, die feuchtigkeitsempfindliche Subtanzen enthalten, offenbart.

Es ist Aufgabe der Erfindung eine weitere Zusammensetzung zu liefern, die eine verbesserte Schleimhautpenetration sowie Anregung der Hormonbildung und insbesondere eine Verbesserung des Allgemeinzustandes bewirkt.

Diese Aufgabe wird erfindungsgemäß durch eine Zusammensetzung der eingangs erwähnten Art dadurch gelöst, das zumindest eine Hormon aus der Gruppe gewählt ist, die aus dem Wachstumshormon Somatotropin (hGH), Erythropoetin (EPO) und humanem Choriogonadotropin (hCG) besteht.

Proteinasen sind Enzyme, die die hydrolytische Spaltung der Peptidbindung im Inneren von Proteinen und Peptiden katalysieren. In der WO 2004/100981 A1 wird eine Zusammensetzung offenbart, die ein oder mehrere Hydrolasen sowie ein oder mehrere Antioxidantien enthält.

Die Polypeptidkette des Hormons wird zunächst in größere und nach kurzer Zeit in kleinere Segmente (Spaltprodukte) mittels der Proteinase bzw. Proteinasenmischung gespalten, um die Aufnahme von biologisch und biochemisch wirksamen Proteinsegmenten langer Polypeptidketten über muköses Gewebe, wie beispielsweise Wangenschleimhaut oder die Nasenschleimhaut zu ermöglichen. Bei den erfindungsgemäß eingesetzten Hormonen handelt es sich bevorzugt um Peptidhormone bzw. Glycoproteinhormone, deren Molekülgröße vor der enzymatischen Spaltung ihre Aufnahme über Schleimhäute verhindert.

Neben wirksamen Polypeptidsegmenten zu einer allgemeinen Verbesserung des Wohlbefindens werden auch solche Spaltprodukte zur Anregung der Hormonproduktion im Körper bereitgestellt, welche aufgrund ihrer Größe (Kleinheit) durch die Schleimhaut aufgenommen werden können.

Durch die Einwirkung der Proteinase auf das Hormon, das üblicherweise aus einer langen Polypeptidkette besteht, wird dieses in Spaltpeptide mit geringerem Molekulargewicht gespalten, und zwar in einem Zeitraum von weniger als eine Minute in Peptidfragmente mit einer Masse von 0,6 bis 6 kD (entspricht 5 bis 50 Aminosäuren), die sodann über das muköse Gewebe vom Körper aufgenommen werden können. Diese Spaltpeptide oder Hormonbruchstücke regen den Körper bzw. die zuständige Drüse an, das ursprünglich in der erfindungsgemäßen Zusammensetzung enthaltene ungespaltene Hormon in erhöhtem Maß zu synthetisieren. So wurde beispielsweise in mehreren Studien (z.B.: in einem Artikel von Dr. Hans-Günter Kugler in der Co'Med 8/2002) festgestellt, dass sich nach Zugabe von L-Glutamin das hGH-Niveau erhöhte, die Proteinsynthese verbesserte und das Immunsystem signifikant verstärkt wurde. Ebenso ist bekannt, dass L-Arginin die hGH-Produktion anregt, in dem es die Absonderung von Somatostatin (einem Wachstumshormon-Hemmer) hemmt und die pituitäre Drüse, die menschliches Wachstumshormon freigibt, anregt, hGH zu produzieren. Somit sind die durch die Spaltung des hGH erhaltenen Peptidfragmente geeignet, die Hormonproduktion anzuregen. Dies kann auch bei anderen Peptidhormonen als dem Somatotropin festgestellt werden. Der Wirkmechanismus der erfindungsgemäßen Zusammensetzung ist hierbei vergleichbar beispielsweise mit einer Impfung, bei welcher Bruchstücke von Viren und/oder Antikörper dem Körper verabreicht werden, um die Bildung von Antikörpern anzuregen.

Während der Lagerung der erfindungsgemäßen Zusammensetzung müssen das Hormon und die Proteinase bzw. Proteinasenmischung voneinander getrennt bleiben, um eine vorzeitige Hydrolyse des Hormons zu vermeiden.

Hierbei ist das zumindest eine Hormon aus der Gruppe gewählt, die ausdem Wachstumshormon Somatotropin (hGH), Erythropoetin (EPO) und humanem Choriogonadotropin (hCG) besteht. Die Unterstützung der Somatotropinsynthese verbessert die Gedächtnisleistung und steigert das Wohlbefinden. Erythropoetin ist ein für die Bildung der roten Blutkörperchen verantwortliches Hormon und wird bei der Behandlung von Anämien, insbesondere in Krebstherapien, verabreicht. Des Weiteren wird es, teilweise im Spitzensport auch auf missbräuchliche Weise, für die Steigerung der körperlichen Leistungsfähigkeit eingesetzt. Choriogonadotropin schließlich ist ein während einer Schwangerschaft vom Körper gebildetes Hormon, das für die Einnistung des Embryos in der Plazenta verantwortlich ist. Eine Unterstützung der Synthese von hCG kann das Risiko einer Fehlgeburt in den ersten Schwangerschaftswochen reduzieren sowie die Fruchtbarkeit erhöhen.

Am Beispiel des Somatotropin wird die Wirkungsweise der erfindungsgemäßen Zusammensetzung erläutert: Die biologisch aktiven Wirkungen des somatotropen Hormons werden nicht vom gesamten Molekül bewirkt und erzielt, sondern von Stellen, die an die DNA direkt andocken können. Diese sogenannten DNA-Response-Elements entfalten dort ihre Wirkung auf die Transskription. Dies betrifft nur gewisse Bereiche im Molekül des Wachstumshormons, während andere Areale für die Stabilität des Moleküls, etc. verantwortlich sind. Aus diesem Grund kann davon ausgegangen werden, dass diese Wirkungsweise auch bei anderen Hormonen gilt, bei welchem ein oder mehrere Fragmente des gesamten Hormons die stimulative Wirkung hervorruft. Wird ein Hormon in kleine, und damit insbesondere über Schleimhäute resorbierbare Fragmente gespalten, so wird wiederum die selbe Wirkung erzielt, weil eben jene Teile des Hormons umfasst oder beinhaltet sind, die die entsprechend Wirkungen an der DNA hervorrufen. Somit wird zwar die Struktur des Hormons gespalten, die Wirkung bleibt hierbei jedoch erhalten, weil Fragmente bei der Spaltung erhalten bleiben, die über aktive Bereiche verfügen, die die gewünschte Wirkung hervorrufen. Es ist zudem nicht auszuschließen, dass durch die Fragmentierung des Hormonmoleküls die Wirkungsweise noch verstärkt wird, weil die kleineren Fragmente aufgrund fehlender räumlicher und/oder sterischer Behinderung rascher zu den und leichter an den Rezeptoren andocken können, so dass tatsächlich eine verstärkte Stimulation der Hormonbildung erzielt wird. Beispielsweise ist bekannt, dass Gerinnungsfaktoren (z.B.: Faktor VII, Faktor VIII) erst durch proteolytische Spaltung aktiviert werden und deren Fragmente die gewünschte Wirkung hervorrufen.

In einer bevorzugten Ausführung der Erfindung ist die Proteinase oder die Proteinasenmischung aus jener Gruppe ausgewählt, die Papain, Bromelain, Ficin und Asclepain enthält.

Papain ist eine aus dem Milchsaft (Latex) unreifer Papayas gewonnene Proteinase, ein kristallines Polypeptid mit einem Molekulargewicht von 23.350 Da, das aus einer Kette von 212 Aminosäure-Resten mit vier Disulfid-Brücken besteht, deren Sequenz und Raumstruktur bekannt sind. Andere Papain-ähnliche pflanzliche Enzyme sind Bromelain (aus Ananas), Ficin (aus Feigen) und Asclepain (aus Asclepia speciosa, ein Seidenpflanzengewächs). Diese Proteinasen sind einfach zu gewinnen und allgemein gut verträglich.

Besonders bevorzugt erfolgt die Aufnahme der Zusammensetzung über die Mundschleimhaut. Dabei treten das zumindest eine Hormon und die zumindest eine Proteinase oder Proteinasenmischung erst in der Mundhöhle in Kontakt miteinander. Das im Mundraum herrschende Milieu gewährleistet eine rasche Spaltung des Hormons innerhalb weniger Sekunden. Die Spaltprodukte werden über die Mundschleimhaut aufgenommen, während die Proteinase und gegebenenfalls nicht abgebautes Hormon aufgrund ihrer Molekülgröße nicht in die Mundschleimhaut eindringen können, geschluckt und im Magen abgebaut werden, ohne schädliche Nebenprodukte freizusetzen.

Es hat sich herausgestellt, dass die rascheste Spaltung des Hormons erfolgt, wenn das zumindest eine Hormon und die zumindest eine Proteinase oder Proteinasenmischung in einem äquimolaren Verhältnis zueinander vorliegen.

Zusätzlich enthält die erfindungsgemäße Zusammensetzung bevorzugterweise pharmazeutisch verträgliche Hilfsstoffe, insbesondere Lösungsmittel, Konservierungsmittel und Aromastoffe, die die Lagerbarkeit sowie die Verabreichbarkeit der Zusammensetzung und den Geschmack verbessern.

In einer bevorzugten Ausführung der Erfindung enthält die Zusammensetzung zusätzlich Vitamine und/oder Spurenelemente, die das Wohlbefinden und die Gesundheit des Anwenders weiter vorteilhaft beeinflussen.

Erfindungsgemäß wird eine erfindungsgemäße Zusammensetzung, die ein Hormon sowie zumindest eine Proteinase oder Proteinasenmischung enthält, als Nahrungsergänzungsmittel zur Verbesserung des geistigen und körperlichen Wohlbefindens eingesetzt. So hat sich herausgestellt, dass die Kombination von Somatotropin mit zumindest einer Proteinase, insbesondere Papain, oder einer Proteinasenmischung aus zwei oder mehreren Proteinasen, Alterungsprozessen entgegenwirkt.

Um eine einfache Verabreichung der erfindungsgemäßen Zusammensetzung über die Mundschleimhaut zu ermöglichen, ist ein Kit bestehend aus einer ersten Spraydose enthaltend zumindest ein Hormon, und einer zweiten Spraydose enthaltend zumindest eine Proteinase oder eine Proteinasenmischung, vorgesehen. Der Anwender muss lediglich zuerst die das oder mehrere Hormone enthaltene Lösung aus der ersten Spraydose und unmittelbar anschließend die Proteinase enthaltende Lösung aus der zweiten Spraydose beispielsweise auf die Wangenschleimhaut sprühen. Dabei können weder das Hormon noch die Proteinase über die Wangeschleimhaut in den Organismus gelangen, während die durch die hydrolytische Spaltung des Hormons mittels der Proteinase oder Proteinasenmischung entstehenden Spaltprodukte aufgrund ihrer geringeren Molekülgröße aufgenommen werden und eine Anregung der Hormonbildung bewirken.

Im Folgenden wird anhand eines Ausführungsbeispiels die Erfindung näher erläutert.

Das erfindungsgemäße Nahrungsergänzungsmittel enthält neben anderen Verbindungen wie beispielsweise Konservierungsstoffe und Aromastoffe, das menschliche Wachstumshormon, nämlich Somatotropin (hGH). Es ist als Lösung in einer ersten Spraydose abgefüllt. In einer zweiten Spraydose befindet sich eine spraybare Lösung, die die Proteinase Papain enthält.

Zunächst wird das Wachstumshormon aus der ersten Spraydose auf die Wangenschleimhaut gesprüht. Anschließend wird die Proteinase aus der zweiten Spraydose durch Sprühen auf die selbe Stelle der Schleimhaut aufgetragen.

Die Zusammensetzung eines Hubs (entspricht in etwa 0,1 ml Sprayflüssigkeit) aus jeder Spraydose ist in der nachfolgenden Tabelle 1 zusammengefasst.

**Tabelle 1:**

| **Spray 1** | | **Spray 2** | |
|---|---|---|---|
| Inhaltsstoffe pro Hub | | Inhaltsstoffe pro Hub | |
| | | | |
| Somatotropin (hGH) | 300ng | Papain | 300ng |
| Aqua purificata | 0,09ml | Aqua purificata | 0,075ml |
| Natriumbenzoat | 0,015mg | Natriumbenzoat | 0,015mg |
| Kaliumsorbat | 0,025mg | Kaliumsorbat | 0,025mg |
| Xylit | 15mg | Passionsfrucht Codi | 2,5mg |
| | | Xanthan FNCS 1165/01,04 | 0,2mg |
| | | Xylit | 15mg |
| | | 96% Ethanol | 10,5mg |

Das Somatotropin wird bei gewöhnlicher oraler Einnahme durch die Magensäfte zerstört und kann somit keine der gewünschten Wirkungen im Körper entfalten. Ebenso ist das Somatotropin-Molekül mit einem mittleren Molekulargewicht von etwa 21.500 Da zu groß, um über die menschliche oder tierische Schleimhaut aufgenommen zu werden.

Es wird daher das Wachstumshormon zunächst auf die Schleimhaut, bevorzugterweise die Mundschleimhaut, aufgetragen und unmittelbar darauf die Proteinase, das Papain aufgesprüht. Die Proteinase hat nun die Aufgabe, das hGH enzymatisch in einzelne Proteinfragmente zu zerlegen, die über die Schleimhaut aufgenommen werden können. Proteinfragmente mit einer Aminosäureanzahl von etwa 80 werden aufgrund ihrer Größe sofort durch die Mundschleimhaut aufgenommen und werden nicht weiter hydrolytisch abgebaut, weil das Papain aufgrund seiner Molekülgröße über die Mundschleimhaut nicht aufgenommen wird.

In einer Versuchsreihe wurde untersucht, inwieweit eine Spaltung des Wachstumshormons Somatotropin durch die Einwirkung von Papain stattfindet. Hierfür wurden die molekularen Massen der durch Papain-Einwirkung in verschiedenen Zeiträumen entstehenden Spalt-Peptide mittels Massenspektrometrie ermittelt.

Die Untersuchungen wurden insgesamt mit drei unterschiedlichen Mischungsverhältnissen von hGH zu Papain durchgeführt. Neben dem äquimolaren Verhältnis (hGH : Papain = 1:1) wurden auch zwei sub-äquimolare Verhältnisse (hGH : Papain = 4:1 bzw. 10:1) untersucht. Als Referenz diente jeweils eine hGH-Probe ohne Zusatz an Papain.

Für die Zeitreihen-Untersuchungen mittels MALDI-TOF-Massenspektrometrie wurden 10 µL der hGH-Stammlösung (mit einer Konzentration von 4 mg/mL) mit 180 µL 20 mM Ammonacetat-Lösung in einem 500 µL fassendes Reaktionsgefäß vermischt. Durch Zugabe von 10 µL der Papain-Stammlösung (mit einer Konzentration von 4 mg/mL) wurde die Reaktion gestartet. Sowohl hGH als auch Papain lagen somit in einer Konzentration von je 0,2 mg/mL vor. Diese Mengen entsprechen einem annähernd äquimolaren Mischungsverhältnis zwischen hGH und Papain.

Die enzymatische Peptidase-Reaktion des Papains wurde nach verschiedenen Zeit-Intervallen gestoppt. Diese Zeitintervalle betrugen 15, 30, 60, 120, 180 und 300 Sekunden. Das Stoppen der Enzymwirkung und der Spaltungsreaktion wurde in Parallelexperimenten auf zwei unterschiedliche Arten durchgeführt.
(1) Spotten der hGH/Papain-Lösung auf das MALDI-Target und sofortige Zugabe von Trifluoressigsäure (TFA). Dabei wurde ein Abbruch der Enzymreaktion durch niedrigen pH-Wert erwartet.
(2) Zugabe von Phenylmethylsulphonylfluorid (PMSF) als Enzym-Inhibitor zur hGH/Papain-Lösung nach einem gewählten Zeitpunkt.

Insgesamt wurden sechs unabhängige Messserien zum Zeitverlauf des enzymatischen Abbaues durchgeführt, die sich aus den angeführten unterschiedlichen Mischungsverhältnissen zwischen hGH und Papain und den verschiedenen Wegen der Enzyminhibition ergaben.

Die Bestimmung der Umsetzungsprodukte erfolgte durch Bestimmung der molekularen Masse der Spaltpeptide mittels MALDI-TOF-MS. Da Papain keine spezifische Spaltstelle besitzt, können aus den Peptidmassen nicht auch die Peptidsequenzen ermittelt werden.

Bei der Untersuchung der entstandenen Spaltpeptide aus den sechs Messreihen mittels Massenspektrometrie wurde Folgendes festgestellt:

hGH wird von Papain bereits innerhalb eines Zeitbereiches von weniger als einer Minute in wässrigen Lösungen unter neutralen pH Bedingungen zu einem erheblichen Anteil in Peptide gespalten, wenn vermutlich auch nicht vollständig. Nach etwa 3 Minuten ist vom ursprünglichen hGH nichts mehr übrig. Infolge der unspezifischen Spaltung von hGH entstehen mehrere Fragmente, die auch innerhalb eines Zeitraumes bis 7 min nicht mehr nennenswert weiter gespalten werden. Ihre molekularen Massen liegen im Bereich von 600 bis 4.500 Da, das entspricht Peptidgrößen von 5 bis etwa 30 Aminosäuren. Diese Muster sind gut reproduzierbar und werden auch durch die Menge der Enzymzugabe (1:1 bzw. 1:4 und 1:10) tendenziell nicht wesentlich verändert.

Diese derart dem Körper zugeführten Proteinfragmente unterstützen den Wachstumshormonaufbau in der Hypophyse, wobei diese Wachstumshormone für das Wohlbefinden, insbesondere für eine Verlangsamung von Alterungsprozessen sowie für die geistige Leistungsfähigkeit, wesentlich sind.

Es hat sich herausgestellt, dass bei regelmäßiger Anwendung der erfindungsgemäßen Zusammensetzung eine Verbesserung der Gedächtnisleistung, des Haarwuchses, eine Verzögerung des Grauwerdens des Haars und allgemein ein höheres körperliches und geistiges Wohlbefinden festgestellt wurde. In einer ersten Untersuchungsreihe wurde festgestellt, dass von 101 Probanden, von welchen 40 Personen ein Plazebo erhielten und somit die Kontrollgruppe bildeten, die Versuchsgruppe mit 31 Männer und 30 Frauen im Alter zwischen 25 und 35 Jahren nach einer Einnahme der erfindungsgemäßen Zusammensetzung von durchschnittlich 9 Monaten insbesondere eine signifikante Steigerung von Ausdauer und Energie, der Produktivität im Alltag, der Sexualfunktionen sowie des Haarund Nagelwachstums feststellte im Vergleich zu der Kontrollgruppe. Ein Unterschied in der Wirkung hinsichtlich des Geschlechts der Probanden konnte hierbei nicht festgestellt werden.

Die erfindungsgemäße Zusammensetzung kann variieren; so kann anstatt Papain ein anderes proteinspaltendes Enzym wie beispielsweise Bromelain, Ficin oder Asclepain oder aber auch Mischungen unterschiedlicher Proteinasen verwendet werden Es können auch Mischungen von Hormonen, beispielsweise EPO mit hCG, vorliegen. Des Weiteren ist die Anwendung nicht auf die Mundschleimhäute beschränkt; die Aufnahme der Spaltpeptide könnte ebenso beispielsweise über die Nasenschleimhäute erfolgen. Auch andere Darreichungsformen anstatt von Sprays sind denkbar. So könnten das Hormon sowie die Proteinase in Form einer Lutschtablette vorliegen, wobei das oder die Hormone und/oder die Proteinase voneinander über eine wasserlösliche Barriere, beispielsweise durch Verkapselung, voneinander getrennt sind und erst beim Lutschen der Tablette freigesetzt werden und miteinander reagieren, wobei die entstehenden Spaltprodukte wiederum über die Mundschleimhaut aufgenommen werden. Ebenso können Kapseln vorgesehen, sein, welche sich im Darm erst lösen.

## Patentansprüche

1. Zusammensetzung zur Aufnahme über muköses Gewebe enthaltend zumindest ein mittels Proteinasen spaltbares Hormon sowie zumindest eine Proteinase oder eine Proteinasenmischung, wobei das zumindest eine Hormon in der Zusammensetzung getrennt von der Proteinase oder Proteinasenmischung vorliegt, **dadurch gekennzeichnet, dass** das zumindest eine Hormon aus der Gruppe gewählt ist, die aus dem Wachstumshormon Somatotropin (hGH), Erythropoetin (EPO) und humanem Choriogonadotropin (hCG) besteht.

2. Zusammensetzung nach Anspruch 1, **dadurch gekennzeichnet, dass** die Proteinase oder die Proteinasenmischung aus der Gruppe, die Papain, Bromelain, Ficin und Asclepain enthält, ausgewählt ist.

3. Zusammensetzung nach Anspruch 1 oder 2, **dadurch gekennzeichnet, dass** das zumindest eine Hormon und die zumindest eine Proteinase in einem äquimolaren Verhältnis zueinander vorliegen.

4. Zusammensetzung nach einem der Ansprüche 1 bis 3, **dadurch gekennzeichnet, dass** sie zusätzlich pharmazeutisch verträgliche Hilfsstoffe, insbesondere Lösungsmittel, Konservierungsmittel und Aromastoffe, enthält.

5. Zusammensetzung nach einem der Ansprüche 1 bis 4, **dadurch gekennzeichnet, dass** sie zusätzlich Vitamine und/oder Spurenelemente enthält.

6. Verwendung einer Zusammensetzung nach einem der Ansprüche 1 bis 5 zur Herstellung eines Nahrungsergänzungsmittels zur Verbesserung des körperlichen und geistigen Wohlbefindens, wobei die Zusammensetzung bevorzugt Somatotropin enthält.

7. Kit zur Verabreichung einer Zusammensetzung nach einem der Ansprüche 1 bis 5 über die Mundschleimhaut bestehend aus einer ersten Spraydose enthaltend zumindest ein Hormon, und einer zweiten Spraydose enthaltend zumindest eine Proteinase oder eine Proteinasenmischung; wobei das Hormon aus der Gruppe gewählt ist, die aus dem WachstumsHormon Somatotropin (hGH), Erythropoietin (EPO) und humanem Choriogonadotropin (hCG) besteht.

## Claims

1. Composition for absorption through mucous tissue containing at least one hormone which can be split by proteinases and at least one proteinase or mixture of proteinases, where the at least one hormone is kept separate from the proteinase or mixture of proteinases in the composition, **characterised in that** the at least one hormone is selected from the group consisting of the growth hormone somatotropin (hGH), erythropoietin (EPO) and human chorionic gonadotropin (hCG).

2. Composition according to claim 1, **characterised in that** the proteinase or mixture of proteinases is selected from the group comprising papain, bromelain, ficin and asclepain.

3. Composition according to any of claims 1 or 2, **characterised in that** the at least one hormone and the at least one proteinase are present in equimolar ratio.

4. Composition according to any of claims 1 to 3, **characterised in that** it additionally contains pharmaceutically well-tolerated auxiliary substances, in particular solvents, preservation agents and flavouring agents.

5. Composition according to any of claims 1 to 4, **characterised in that** it additionally contains vitamins and/or trace elements.

6. Use of a composition according to any of claims 1 to 5 for the production of a nutritional supplement for improving physical and mental well-being, said composition preferably containing somatotropin.

7. Kit for administering a composition according to any of claims 1 to 5 via the mucous membrane of the mouth, comprising a first spraycan containing at least one hormone and a second spraycan containing at least one proteinase or mixture of proteinases, where the hormone is selected from the group consisting of the growth hormone somatotropin (hGH), erythropoietin (EPO) and human chorionic gonadotropin (hCG).

## Revendications

1. Composition destinée à être appliquée sur un tissus muqueux renfermant au moins une hormone pouvant être clivée avec des protéinases ainsi qu'au moins une protéinase ou un mélange de protéinases, la où les hormone(s) étant séparée(s) de la protéinase ou du mélange de protéinases dans la composition,
**caractérisée en ce que**
la ou les hormone(s) est (sont) choisie(s) dans le groupe formé par l'hormone de croissance somatotropine (hGH), l'érythroproétine (EPO) et la choriogonadotropine humaine (hCG).

2. Composition conforme à la revendication 1,
**caractérisée en ce que**
la protéinase ou le mélange de protéinases est choisi dans le groupe renfermant la papaïne, la bromélaïne, la ficine et l'asclépaïne.

3. Composition conforme à la revendication 1 ou 2,
**caractérisée en ce que**
la ou les hormone(s) et la ou les protéinase(s) sont dans un rapport équimolaire.

4. Composition conforme à l'une des revendications 1 à 3,
**caractérisée en ce qu'**
elle renferme en outre des additifs pharmaceutiquement acceptables, en particulier des solvants, des agents de conservation et des agents aromatiques.

5. Composition conforme à l'une des revendications 1 à 4,
**caractérisée en ce qu'**
elle renferme en outre des vitamines et/ou oligoéléments.

6. Utilisation d'une composition conforme à l'une des revendications 1 à 5 pour l'obtention d'un complément alimentaire pour améliorer le bien être corporel et spirituel, cette composition renfermant de préférence de la somatotropine.

7. Kit pour l'administration d'une composition conforme à la revendication 1, sur la mucosité buccale constitué par une première dose à pulvériser renfermant au moins une hormone et par une seconde dose à pulvériser renfermant au moins une protéinase ou un mélange de protéinases, l'hormone étant choisie dans le groupe formé par l'hormone de croissance somatotropine (hGH), l'érythroproétine (EPO) et la choriogonadotropine humaine (hCG).
